# EUROPÄISCHE PATENTSCHRIFT

(11) **EP 1 136 487 B1**
(45) Veröffentlichungstag und Bekanntmachung des Hinweises auf die Patenterteilung: **25.05.2005**
(21) Anmeldenummer: 01105450.9
(22) Anmeldetag: 14.03.2001
(51) Int. Cl.: C07D 401/04, C07D 291/06, C07D 275/06, A23L 1/236, A61K 31/4427, A61K 9/00, A61K 31/54, A24B 15/00

(54) **Nikotinsalze mit verbessertem Geschmack, Verfahren zu ihrer Herstellung und ihre Verwendung als Antirauchmittel**
Nicotine salts with improved taste, process for their preparation and use thereof as smoking cessation agents
Sels de la nicotine, procédé pour leur préparation et leur utilisation comme agents pour la cessation de fumer

(30) Priorität: 20.03.2000 DE 10013712
(43) Veröffentlichungstag der Anmeldung: 26.09.2001
(73) Patentinhaber: Nutrinova Nutrition Specialties & Food Ingredients GmbH, 65929 Frankfurt am Main (DE)
(72) Erfinder: Burgard, Andreas, Dr., 65929 Frankfurt am Main (DE); Dörr, Margit, 67591 Hohen-Sülzen (DE)
(74) Vertreter: Schweitzer, Klaus, Dr.

(56) Entgegenhaltungen:
- WO-A-00/12067
- WO-A-00/35296
- WO-A-97/41843
- WO-A-99/04822
- US-A- 5 488 962
- CHEMICAL ABSTRACTS, vol. 101, no. 19, 5. November 1984 (1984-11-05) Columbus, Ohio, US; abstract no. 167543, TRENKER, ADY: "Sucking composition for stopping smoking" XP002170271 & BE 899 037 A (BELG.) 18. Juni 1984 (1984-06-18)

## Beschreibung

Raucher gewöhnen sich durch kontinuierlichen Konsum von Zigaretten daran, regelmäßig Nikotin zu sich zu nehmen. Aufgrund der nachteiligen Auswirkungen des Rauchens auf die Gesundheit sind viele Raucher daran interessiert, das Rauchen aufzugeben.

Dieses Vorhaben scheitert in vielen Fällen aber daran, daß die regelmäßige Aufnahme von Nikotin bereits zu einer körperlichen Abhängigkeit geführt hat. Für eine langsame Entwöhnung sind verschiedene Wege vorgeschlagen worden, zum Beispiel Nikotin in Form imprägnierter Pflaster auf die Haut aufzubringen oder es oral z. B. über ein Kaugummi zuzuführen (US 5,488,962). Die Zufuhr über Kaugummi hat zusätzlich den Vorteil, daß die orale Aufnahme das Bedürfnis, an Zigaretten zu saugen, kompensiert.

Nikotin besitzt allerdings einen Geschmack, der bei alleiniger Aufnahme als unangenehm empfunden wird. Er wird in der Regel als scharf bis pfefferartig und bitter bezeichnet. Dieser unangenehme Geschmack beeinträchtigt die Akzeptanz von Kaugummi mit Nikotinzusatz, das zur Entwöhnung von Rauchen besonders in der Anfangsphase regelmäßig gekaut werden sollte. Um die Akzeptanz von Nikotin enthaltenden Kaugummis oder anderen Zubereitungen, die oral aufgenommen werden können, zu verbessern, besteht deshalb das Bedürfnis, den unangenehmen Geschmack des Nikotins zu verbessern oder zu maskieren.

Es hat eine ganze Reihe von Versuchen gegeben, den unangenehmen Geschmack des Nikotins zu überdecken. Der Einsatz von Aroma- und Geschmacksstoffen wie Pfefferminzoder Erdbeeraromen führte bisher nicht zu befriedigender Maskierung des Nikotingeschmacks. Lediglich die Verwendung von Einschlußverbindungen, wie z.B. mit Cyclodextrinen (WO 97/41843) ist bisher geeignet, eine geschmackliche Verbesserung herbeizuführen. Solche Einschlußverbindungen führen allein aber nicht zu ausreichender geschmacklicher Akzeptanz, es müssen vielmehr zusätzliche Geschmacksstoffe, z.B. bei Kaugummi, verwendet werden.

Es wurde deshalb von den Herstellern von Nikotin-Kaugummis bereits versucht, den unangenehmen Geschmack des Nikotins durch Kombination mit Süßstoffen, meist Saccharin und/oder Acesulfam-K zu überdecken, die in die Kaugummigrundmasse eingearbeitet werden. Die Süßstoffe als solche können jedoch den Nikotingeschmack nicht maskieren, so daß der Nikotingeschmack während des Kauvorganges immer noch deutlich wahrnehmbar und mit jedem Kauvorgang deutlicher hervortritt.

Dieser Geschmackseindruck der oben genannten Mischungen kann durch einfache sensorische Versuche mit wäßrigen Lösungen nachvollzogen werden, da sowohl Nikotin als auch die Süßstoffe wasserlöslich sind. Dazu werden Lösungen von Nikotin mit verschiedenen Süßstoffen wie Acesulfam-K, Saccharin-Natrium oder Aspartam in Wasser hergestellt und einer Geschmacksprobe im Vergleich zu einer nikotinhaltigen, wäßrigen Lösung (10 mg Nikotin in 100 ml Wasser) unterzogen. Die verschiedenen Kombinationen von Nikotin mit den jeweiligen Süßstoffen und die entsprechenden Geschmackseindrücke sind in Tabelle 1 dargestellt.

**Tabelle 1**

| Geschmackliche Prüfung der Kombination von Nikotin mit Süßstoffen | | | | | |
|---|---|---|---|---|---|
| **Substanzen** | **mg gelöste Substanz in 100 ml Wasser** | | | | |
| Nikotin | 10 | 10 | 10 | 10 | 10 |
| Acesulfam-K | - | 12 | 24 | 12 | 12 |
| Saccharin-Na | - | - | - | 15 | - |
| Aspartam | - | - | - | | 15 |
| **Geschmack** | sofort pfefferartiger, kratziger, langanhaltender unangenehm bitterer Geschmack | sofort pfefferartiger, kratziger, lang anhaltender unangenehm bitterer Geschmack mit begleitendem leichtem Süßgeschmack | sofort pfefferartiger, kratziger, langanhaltender unangenehm bitterer Geschmack mit begleitendem stärkerem Süßgeschmack | sofort pfefferartiger, kratziger, langanhaltender unangenehm bitterer Geschmack mit bitterem und süßem Nachgeschmack | sofort pfefferartiger, kratziger, langanhaltender unangenehm bitterer Geschmack mit begleitendem leichtem Süßgeschmack |

Die Ergebnisse belegen, daß der unangenehme Geschmack des Nikotins durch einfachen Zusatz von den oben genannten Süßstoffen nicht wesentlich modifiziert werden kann. Deshalb besteht nach wie vor Bedarf, den unangenehmen Geschmack des Nikotin in Zubereitungen, die oral aufgenommen werden, insbesondere in Kaugummi während des Kauvorganges zu maskieren.

Die WO 99/04822 beschreibt 1:1 Salze aus einem Süßstoff und einem unangenehm schmeckenden Pharmazeutikumn. Salze aus einem anionischen Süßstoff und Nikotin werden nicht beschrieben.

Die WO 00/12067 beschreibt 1:1 Salze aus Saccharin mit synthetischen nicht-alkaloidalen medizinischen organischen Basen. Salze aus einem anionischen Süßstoff und Nikotin werden nicht beschrieben.

Die EP-B 0 046 506 beschreibt Vincamin-saccharinat und dieses enthaltene Arzneimittel. Salze aus einem anionischen Süßstoff und Nikotin werden nicht beschrieben.

Es wurde nun gefunden, daß Nikotin aufgrund seines basischen Charakters mit anionenbildenden Süßstoffen, die in Form ihrer korrespondierenden Säure vorliegen, zu salzartigen Verbindungen reagieren kann.

Dabei gelingt nicht allein die Herstellung von Salzen aus einem Molekül Nikotin und einem Molekül Süßstoffen, sondern es lassen sich ebenso definierte Verbindungen aus einem Molekül Nikotin und zwei Molekülen Süßstoff herstellen, wobei sogar zwei verschiedene Süßstoffe eingesetzt werden können. Überraschenderweise zeichnen sich alle diese Verbindungen durch einen angenehmen Süßgeschmack aus, dem die unangenehmen Komponenten des Nikotins weitgehend fehlen. Sie bestehen lediglich aus Komponenten, die z.B. in nikotinhaltigen Kaugummis ohnehin verwendet werden und machen weitere Arbeitsschritte wie die Herstellung der in der WO 97/41843 genannten Einschlußverbindungen überflüssig. Damit stellt diese Erfindung einen wesentlichen Fortschritt in der Herstellung, Zusammensetzung und Verwendung nikotinhaltiger Präparate zur Raucherentwöhnung dar.

Die vorliegende Erfindung betrifft somit Verbindungen oder Salze aus Nikotin und anionenbildenden Süßstoffen oder deren physiologisch verträglichen Salzen oder Süßstoffen, die in freier Form Säurecharakter besitzen, oder deren physiologisch verträglichen Salzen. Diese Verbindungen können auch als sog. Säureadditionssalze, bevorzugt mit HCI, oder anderen physiologisch verträglichen Säuren wie z.B. Essigsäure oder Schwefelsäure vorliegen.

Für die Herstellung dieser Verbindungen eignen sich grundsätzlich alle Süßstoffe oder deren physiologisch verträglichen Salze, wie insbesondere das Kaliumsalz des Acesulfams (Acesulfam-K), die Anionen zu bilden vermögen, dazu gehören insbesondere
Acesulfam und andere Oxathiazinonsüßstoffe
Alitam
Aspartam und aspartamähnliche Di- und Tripeptide
Cyclamat und andere Sulfamatsüßstoffe
Glycyrrhizin
Neotam
Saccharin
Gluconsäure

Acesulfam (Acesulfam -H) und Acesulfam-K sind besonders bevorzugt.

Dabei sind eine Vielzahl von Kombinationen, insbesondere bei den Verbindungen aus einem Molekül Nikotin und zwei Süßstoffmolekülen möglich. Durch geeignete Wahl der Süßstoffe läßt sich, insbesondere in Verbindungen mit zwei verschiedenen Süßstoffen überraschenderweise der Süßgeschmack, insbesondere die zeitliche Wahrnehmung der Süße modifizieren, zum einen im Hinblick auf die Maskierung des Nikotingeschmacks, zum anderen zur Einstellung eines optimalem Gesamtgeschmackseindrucks. Dies ist ein entscheidender Vorteil gegenüber den 1:1 Verbindungen aus 1 Mol Süßstoff und 1 Mol Nikotin.

Die vorliegende Erfindung betrifft somit auch ein Verfahren zur Herstellung der erfindungsgemäßen Nikotin-Süßstoffverbindungen durch Umsetzung von Nikotin mit Süßstoffen in Form ihrer freien Säuren (z.B. Acesulfam-H) in einem geeigneten Lösungsmittel. Geeignete Lösungsmittel sind bevorzugt Wasser und/oder mit Wasser mischbare Lösungsmittel wie z.B. Alkohole. Für diese direkte Umsetzung geeignete Süßstoffe sind bevorzugt Acesulfam, Cyclamat, Glycyrrhizin, Gluconsäure und/oder Saccharin. Eine Verfahrensvariante besteht darin, daß man die Süßstoffe oder deren Salze (z.B. Acesulfam-K) mit Nikotin in einem wie oben beschriebenen Lösungsmittel in Gegenwart einer physiologisch unbedenklichen Säure, bevorzugt Salzsäure, oder anderen geeigneten anorganischen oder organischen Säuren wie z.B. Essigsäure oder Schwefelsäure umsetzt und die resultierenden Säureadditionssalze (vgl. z.B. Schema 1) der Nikotin-Süßstoffverbindungen isoliert.

Die Ausgangsstoffe für die Herstellung der erfindungsgemäßen Verbindungen sind im Handel erhältlich oder können nach literaturbekannten Methoden hergestellt werden; z.B. Acesulfam/Acesulfam-K vgl. EP-A 0 155 643.

Die Darstellung der Nikotin-Süßstoff-Verbindungen, Salze oder Addukte gelingt sehr einfach aus Lösungen, bevorzugt aus wässrigen Lösungen, des Nikotins und der entsprechenden Säure des jeweiligen Süßstoffes oder z.B. in einersalzsauren Lösung des jeweiligen Süßstoffs, wie in den folgenden Beispielen insbesondere für Acesulfam-H gezeigt wird. Die erhaltenen Reaktionslösungen werden in geeigneter Weise, z. B. im Vakuum vom Lösungsmittel befreit. Die Nikotin-Süßstoff-Verbindungen liegen laut ¹H-NMR als 1:1-, bevorzugt als 1:2- bzw. 1:1:1-Addukte vor. Für die Herstellung von Verbindungen ohne zusätzlichen Salzgehalt (keine Säureadditionssalze) werden vorzugsweise die Süßstoffe eingesetzt, die in freier Form Säuren sind.

Die Erfindung betrifft weiterhin die Verwendung der genannten Verbindungen zur Herstellung von festen oder flüssigen Zubereitungen, die zur oralen Zufuhr von Nikotin, bevorzugt in Form von Kaugummi, Kautabletten, Komprimaten oder ähnlichen Zubereitungen, geeignet sind. Weiterhin werden allgemein Zubereitungen, die die erfindungsgemäßen Verbindungen enthalten, von der Erfindung umfaßt.

Die nachfolgenden Beispiele sollen die Erfindung näher erläutern, ohne dabei deren Umfang einzuschränken.

### Beispiel 1

### Herstellung eines 1:1-Salzes aus Nikotin und Acesulfam-H (vgl. Schema 1)

In 20 ml Wasser werden 20 mmol (3,244 g) Nikotin gelöst und mit 20 mmol (3,263 g) Acesulfam-H versetzt. Das Reaktionsgemisch wird anschließend im Vakuum eingeengt. Es resultiert mit 100 % Ausbeute ein gelbbraungefärbtes Öl, welches laut ¹H-NMR als 1:1-Addukt vorliegt.

### Beispiel 2

### Herstellung eines 1:1:1-Salzes aus Nikotin, Acesulfam-H und Saccharin-H (vgl. Schema 2)

In 20 ml Wasser werden 20 mmol (3,244 g) Nikotin gelöst und mit 20 mmol (3,263 g) Acesulfam-H versetzt. Anschließend werden noch 20 mmol (3,664 g) Saccharin-H hinzugefügt. Das Reaktionsgemisch wird anschließend im Vakuum eingeengt. Es resultiert mit 100 % Ausbeute ein gelbbraungefärbtes Öl, welches laut ¹H-NMR als 1:1:1-Addukt vorliegt.

### Beispiel 3

### Herstellung eines 1:2-Salzes aus Nikotin und Acesulfam-H (vgl. Schema 2)

In 20 ml Wasser werden 20 mmol (3,244 g) Nikotin gelöst und mit 40 mmol (6,526 g) Acesulfam-H versetzt. Das Reaktionsgemisch wird anschließend im Vakuum eingeengt. Es resultiert mit 100 % Ausbeute ein gelbgefärbter amorpher Feststoff, welcher laut ¹H-NMR als 1:2-Addukt vorliegt.

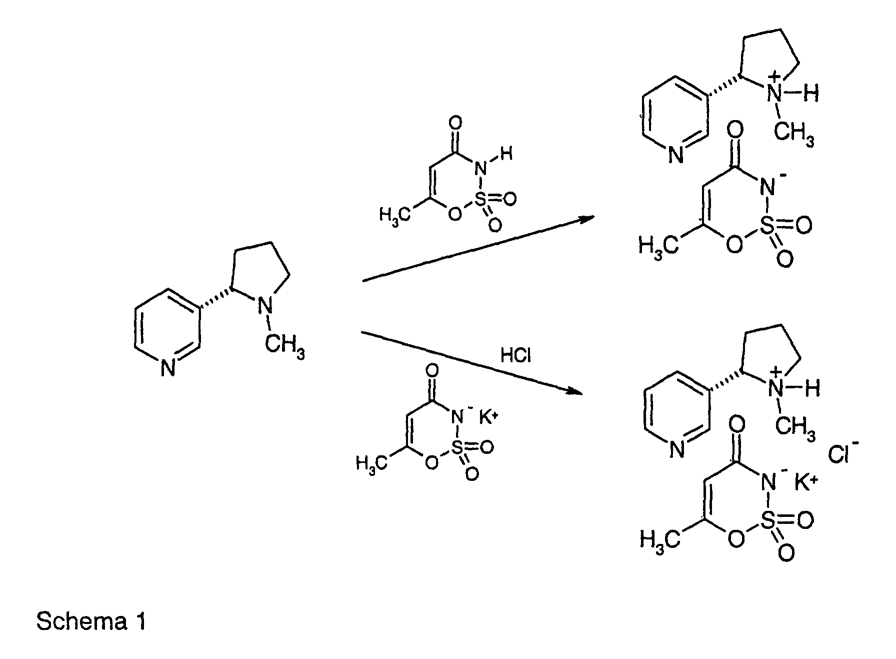

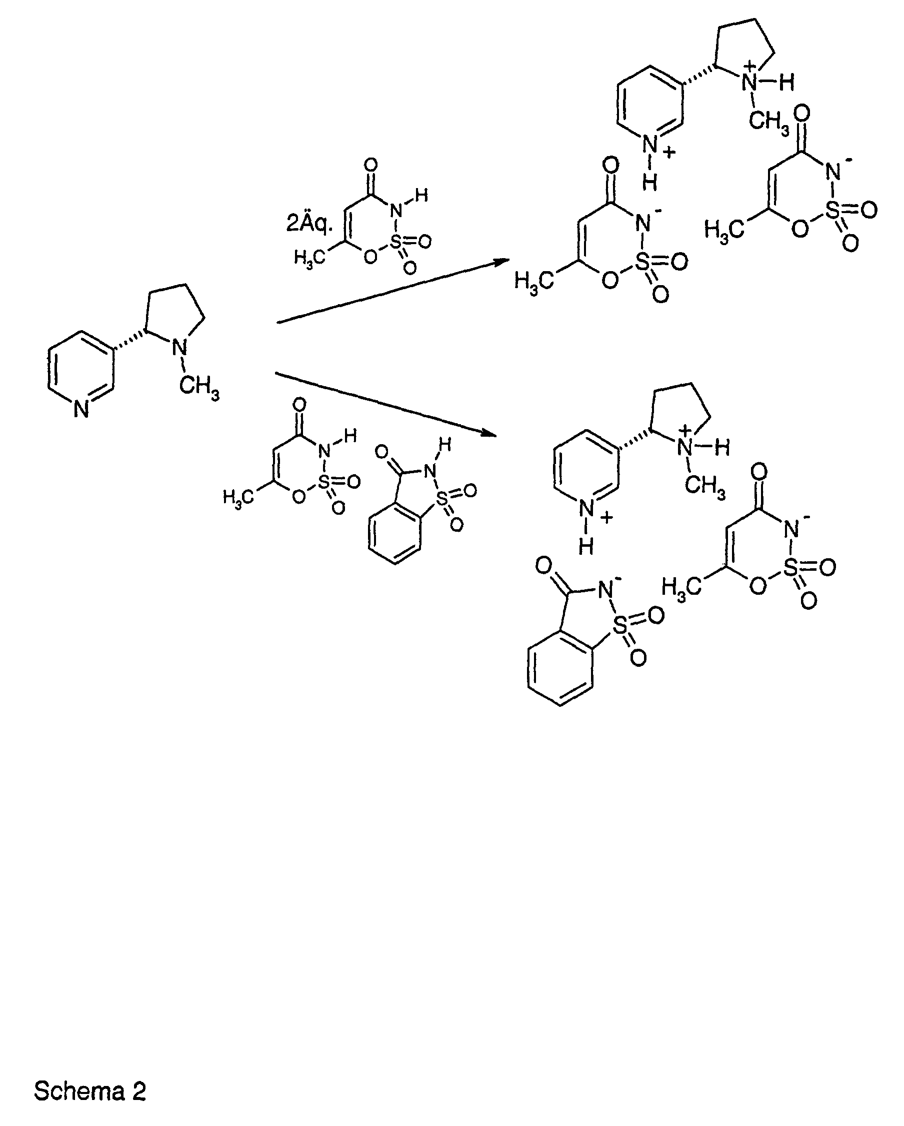

Die verschiedenen Nikotin-Süßstoff-Verbindungen im molaren Verhältnis 1:1 und 1:2 bzw. 1:1:1 wurden in einem Geschmackstest im Vergleich zu einer wäßrigen Nikotinlösung (10 mg Nikotin in 100 ml Wasser) sensorisch untersucht. Beispiel 4 zeigt die Ergebnisse solcher Prüfungen.

### Beispiel 4

### Nikotin-Süßstoff-Verbindungen im Verhältnis 1:1 und 1:2 bzw. 1:1:1

| | **Verbindung** | | | |
|---|---|---|---|---|
| **mg gelöstes Addukt in 100 ml Wasser** | 1:1 Nikotin-Acesulfam-Salz 20 | 1:2 Nikotin-Acesulfam- Salz 31 | 1:1:1 Nikotin-Acesulfam- Saccharin-Salz 35 | 1:1:1 Nikotin-Acesulfam- Aspartam-Salz 66 |
| **Geschmack** | sofort süß, nach etwa 10 Sekunden paprikaartiger Geschmack, anschließend pfefferartig mild | sofort einsetzende deutliche Süße, lang anhaltend, anfangs sauer, kein Nikotingeschmack, nach 5 Minuten: kein Nikotingeschmack | sofort einsetzende, schwache Süße, metallischer, etwas bitterer Bei- und Nachgeschmack, nach 5 Minuten: kein Nikotingeschmack | sofort einsetzende, starke Süße, lang anhaltend, nach 5 Minuten: kein Nikotingeschmack |

Die Ergebnisse belegen, daß, im Gegensatz zur einfachen Mischung des freien Nikotins mit Süßstoffen, der unangenehme Nikotingeschmack durch eine Salz- oderAdduktbildung des Nikotins mit Süßstoffen maskiert werden kann.

Während bei den 1:1-Addukten der Geschmack des Nikotins nach einer längeren Verweilzeit im Mund wieder erkennbar ist, kann der Nikotingeschmack bei den 1:2- bzw. 1:1:1-Addukten des Nikotins mit den jeweiligen Anionen der Süßstoffe überraschenderweise sogar gänzlich beseitigt werden, so daß selbst nach einigen Minuten Verweilzeit im Mund kein Nikotingeschmack feststellbar ist.

Die erfindungsgemäßen Verbindungen sind stabil und zerfallen auch bei der Einarbeitung in Kaugummi oder anderen zur Raucherentwöhnung geeignete Zubereitungen nicht.

Insbesondere können sie in Zwischen- oder Vorprodukten eingesetzt werden, ohne daß sich Nikotin und Süßstoffe entmischen. Sie lassen sich deshalb problemlos in die Zubereitungen einarbeiten, mit denen sie aufgenommen werden sollen, z. B. Kaugummi, Kautabletten, Komprimate oder andere Zubereitungen zur oralen Verwendung.

Die Anwendung wird durch die folgenden Beispiele erläutert.

### Beispiel 5

### Einarbeitung in Kaugummi

Zur Einarbeitung der erfindungsgemäßen Verbindungen in Kaugummi werden zuckerfreie Kaugummi-Streifen aus den allgemein bekannten Zutaten Kaumasse, zuckerfreien Süßungsmitteln wie Zuckeralkohole und Süßstoffe, Glycerin und Aromastoffen und den erfindungsgemäßen Verbindungen nach allgemein bekannter Technologie hergestellt, d.h. die Zutaten werden nacheinander in die erwärmte Kaumasse eingetragen und gleichmäßig eingearbeitet. Anschließend wird wie üblich ausgeformt und portioniert. Die Zutaten entsprechen den üblicherweise verwendeten, d.h. bei Zuckeralkoholen z.B. Sorbit, Xylit, Mannit, Maltit, Isomalt, Laktit, Erythrit, Mischungen aus Zuckeralkoholen und Zuckeralkohole in Sirupform, wie z.B. Sorbit-Sirup und Maltitsirup. Falls erwünscht, können zur zusätzlichen Aufsüßung der Kaugummis alle bekannten Süßstoffe eingesetzt werden, wie z.B. Acesulfam-K, Aspartam, Cyclamat, Saccharin, Thaumatin, Neohesperidine DC, Sucralose, Brazzein, Neotame.

Die Dosage der Nikotinsalze ist abhängig von der Menge an Nikotin, die in einem Streifen Kaugummi enthalten sein soll. Damit z.B. 2 mg Nikotin in einem Kaugummi-Streifen mit 3 g Masse enthalten ist, müssen 6 mg des beschriebenen 1:2 Salzes aus Nikotin und Acesulfamsäure eingesetzt werden oder aber 7,61 mg des beschriebenen Nikotin-Acesulfamsäure-Aspartam-Salzes. Analog kann die Dosage des Nikotin durch Erhöhung der Dosage der Nikotin-Salze erhöht werden.

Die Nikotin-Salze können ebenso in zuckerhaltige Kaugummi-Rezepturen eingearbeitet werden. Hierfür werden anstelle oder gemeinsam mit den Zuckeraustauschstoffen die üblicherweise verwendeten Zuckerstoffe wie z.B. Saccharose und Glukosesirup eingesetzt. Die so erhaltenen Kaugummi weisen einen angenehmen Süßgeschmack auf, der auch bei längerem Kauen nicht von bitteren Geschmackskomponenten abgelöst wird.

### Beispiel 6

### Einarbeitung in Komprimate, Tabletten oder Kautabletten

Zur Einarbeitung der erfindungsgemäßen Verbindungen in Komprimate werden zuckerfreie oder zuckerhaltige Komprimate, Tabletten oder Kautabletten unter Verwendung von zuckerfreien Süßungsmitteln wie Zuckeralkohole und Süßstoffe, Hilfsstoffen, Bindemitteln und Aromastoffen und den erfindungsgemäßen Verbindungen nach allgemein bekannter Technologie hergestellt, d.h. die Zutaten werden nacheinander homogen vermischt und zu Komprimaten verpresst oder Tabletten geformt. Die Zutaten entsprechen den üblicherweise verwendeten, d.h. bei Zuckeralkoholen z.B. Sorbit, Xylit, Mannit, Maltit, Isomalt, Laktit und Erythrit und Mischungen aus Zuckeralkoholen. Bei zuckerhaltigen Komprimaten und Tabletten werden entweder ausschließlich oder in Kombination mit Zuckeralkoholen Zuckerstoffe wie z.B. Saccharose verwendet. Zur zusätzlichen Aufsüßung der Komprimate und Tabletten können alle bekannten Süßstoffe eingesetzt werden, wie z.B. Acesulfam-K, Aspartam, Cyclamat, Saccharin, Thaumatin, Neohesperidine DC, Sucralose, Brazzein, Neotame. Die Dosage der Nikotinsalze ist abhängig von der Menge an Nikotin, die in einem Komprimat oder einer Tablette enthalten sein soll. Zur Dosage von 2 mg Nikotin müssen in ein Komprimat oder in eine Tablette 6 mg des beschriebenen 1:2 Salzes aus Nikotin und Acesulfamsäure eingesetzt werden oder aber 7,61 mg des beschriebenen Nikotin-Acesulfamsäure-Aspartam-Salzes. Analog kann die Dosage des Nikotin durch Erhöhung der Dosage der Nikotin-Salze erhöht werden.

Das so erhaltene Komprimat bzw. die Tablette weist einen angenehmen Süßgeschmack auf, der auch bei längerem Lutschen oder Kauen nicht von bitteren Geschmackskomponenten abgelöst wird.

## Patentansprüche

1. Salz aus Nikotin, anionenbildenden Sußstoff oder anionenbildenden Süßstoffen und/oder deren physiologisch verträglichen Salzen und gegebenenfalls einer physiologisch verträglichen Säure.

2. Salz nach Anspruch 1, **dadurch gekennzeichnet, daß** Nikotin und Süßstoff im molaren Verhältnis von 1 : 1 oder 1 : 2 vorliegen, wobei die Süßstoffmoleküle gleich oder verschieden sein können.

3. Salz nach Anspruch 1 oder 2, **dadurch gekennzeichnet, daß** als Süßstoff Acesulfam oder andere Oxathiazinonsüßstoffe, Alitam, Aspartam oder aspartamähnliche Di- und Tripeptide, Cyclamat oder andere Sulfamatsüßstoffe, Glycyrrhizin, Neotam, Saccharin oder Gluconsäure oder deren physiologisch verträglichen Salze verwendet werden.

4. Salz nach einem oder mehreren der Ansprüche 1 bis 3, **dadurch gekennzeichnet, daß** als Süßstoff Acesulfam oder Acesulfam-K allein oder in Kombination mit einem weiteren Süßstoff oder mit dessen physiologisch verträglichen Salz verwendet wird.

5. Verfahren zur Herstellung eines Salzes nach einem oder mehreren der Ansprüche 1 bis 4, indem man
a) in einem geeigneten Lösungsmittel Nikotin mit einem oder 2 Süßstoffen, die gleich oder verschieden sein können, in Form ihrer freien Säure umsetzt und das gebildete Reaktionsprodukt gegebenenfalls isoliert, oder
b) in einem geeigneten Lösungsmittel Nikotin mit einem oder 2 Süßstoffen, die gleich oder verschieden sein können, oder mit deren physiologisch verträglichen Salzen in Gegenwart einer physiologisch verträglichen Säure umsetzt und das gebildete Reaktionsprodukt gegebenenfalls isoliert.

6. Verfahren nach Anspruch 5, **dadurch gekennzeichnet, daß** Wasser und/oder mit Wasser mischbare Lösungsmittel als Lösungsmittel verwendet werden.

7. Verfahren nach Anspruch 5 oder 6, **dadurch gekennzeichnet, daß** als physiologisch verträgliche Säure Salzsäure verwendet wird.

8. Verwendung einer eines Salzes nach einem oder mehreren der Ansprüche 1 bis 4 zur Herstellung von festen oder flüssigen Zubereitungen, bevorzugt in Form von Kaugummi, Kautabletten oder Komprimaten, zur oralen Verabreichung von Nikotin.

9. Feste oder flüssige Zubereitung, bevorzugt in Form von Kaugummi, Kautabletten oder Komprimaten, zur oralen Verabreichung von Nikotin, **dadurch gekennzeichnet, daß** sie ein Salz nach einem oder mehreren der Ansprüche 1 bis 4 enthält.

## Claims

1. A salt of nicotine, anion-forming sweetener or anion-forming sweeteners and/or their physiologically acceptable salts and, if appropriate, a physiologically acceptable acid.

2. A salt as claimed in claim 1, wherein nicotine and sweetener are present in a molar ratio of 1:1 or 1:2, in which case the sweetener molecules can be identical or different.

3. A salt as claimed in claim 1 or 2, wherein the sweetener used is acesulfame or other oxathiazinone sweeteners, alitame, aspartame or aspartame-like dipeptides and tripeptides, cyclamate or other sulfamate sweeteners, glycyrrhizin, neotame, saccharin or gluconic acid or their physiologically acceptable salts.

4. A salt as claimed in one or more of claims 1 to 3, wherein the sweetener used is acesulfame or acesulfame-K alone or in combination with a further sweetener or with its physiologically acceptable salt.

5. A process for producing a salt as claimed in one or more of claims 1 to 4, by
a) reacting, in a suitable solvent, nicotine with one or 2 sweeteners, which can be identical or different, in the form of their free acid and if appropriate isolating the reaction product formed, or
b) reacting, in a suitable solvent, nicotine with one or 2 sweeteners, which can be identical or different, or with their physiologically acceptable salts in the presence of a physiologically acceptable acid, and if appropriate isolating the reaction product formed.

6. The process as claimed in claim 5, wherein water and/or water-miscible solvents are used as solvent.

7. The process as claimed in claim 5 or 6, wherein the physiologically acceptable acid used is hydrochloric acid.

8. The use of a salt as claimed in one or more of claims 1 to 4 for producing solid or liquid preparations, preferably in the form of chewing gum, chewing tablets or compressed compositions, for the oral administration of nicotine.

9. A solid or liquid preparation, preferably in the form of chewing gum, chewing tablets or compressed compositions, for the oral administration of nicotine, wherein it comprises a salt as claimed in one or more of claims 1 to 4.

## Revendications

1. Sel de nicotine, d'un ou de plusieurs édulcorants formant des anions et/ou de leurs sels physiologiquement acceptables et le cas échéant d'un acide physiologiquement acceptable.

2. Sel selon la revendication 1, **caractérisé en ce que** la nicotine et l'édulcorant sont présents en ratio molaire de 1 :1 ou 1 :2, les molécules d'édulcorant pouvant être identiques ou différents.

3. Sel selon la revendication 1 ou 2, **caractérisé en ce qu'**est mis en oeuvre à titre d'édulcorant l'acesulfam ou d'autres édulcorants oxathiazinone, alitame, aspartame ou des di- ou tripeptides similaires à l'aspartame, cyclamate ou d'autres édulcorants sulfamate, glycyrrhizine, néotame, saccharine ou acide gluconique ou leurs sels physiologiquement acceptables.

4. Sel selon l'une quelconque des revendications 1 à 3, **caractérisé en ce qu'**est utilisé à titre d'édulcorant l'acésulfam ou l'acésulfam-K, seul ou en combinaison avec un autre édulcorant ou avec son sel physiologiquement acceptable.

5. Procédé de préparation d'un sel selon l'une quelconque des revendications 1 à 4, dans lequel
a) la nicotine est laissée réagir avec un ou deux édulcorants identiques ou différents sous forme de leurs acides libres dans un solvant approprié et le cas échéant le produit réactionnel formé est isolé, ou
b) la nicotine est laissée réagir avec un ou deux édulcorants identiques ou différents, ou avec leurs sels physiologiquement acceptables, en présence d'un acide physiologiquement acceptable dans un solvant approprié, et le cas échéant le produit réactionnel formé est isolé.

6. Procédé selon la revendication 5, **caractérisé en ce que** l'eau et/ou des solvants miscibles à l'eau sont utilisés à titre de solvant.

7. Procédé selon la revendication 5 ou 6, **caractérisé en ce que** l'acide chlorhydrique est utilisé à titre d'acide physiologiquement acceptable.

8. Utilisation d'un sel selon l'une quelconque des revendications 1 à 4, pour la préparation de compositions solides ou liquides, de préférence sous forme de gommes à mâcher, comprimés à mâcher ou comprimés, pour l'administration orale de nicotine.

9. Composition solide ou liquide, de préférence sous forme de gommes à mâcher, comprimés à mâcher ou comprimés, pour l'administration orale de nicotine, **caractérisée en ce qu'**elle contient un sel selon l'une quelconque des revendications 1 à 4.
